(19) Europäisches Patentamt
European Patent Office
Office européen des brevets

(11) Veröffentlichungsnummer : **0 049 498**
**B1**

(12) # EUROPÄISCHE PATENTSCHRIFT

(45) Veröffentlichungstag der Patentschrift : **30.01.85**

(21) Anmeldenummer : **81107865.8**

(22) Anmeldetag : **02.10.81**

(51) Int. Cl.[4] : **C 07 F 5/06**, A 61 K 7/38// C07C53/126, C07C57/32, C07C63/08, C07C63/70, C07C65/21, C07C69/92, C07C69/773, C07C31/32, C07C29/68, C07D213/80

(54) **Organische Reste enthaltende Aluminiumverbindungen, Transpiration hemmende Mittel, die diese Verbindungen als Wirkstoffe enthalten sowie Verwendung dieser Mittel zur Hemmung der Transpiration.**

(30) Priorität : **03.10.80 CH 7403/80**

(43) Veröffentlichungstag der Anmeldung : **14.04.82 Patentblatt 82/15**

(45) Bekanntmachung des Hinweises auf die Patenterteilung : **30.01.85 Patentblatt 85/05**

(84) Benannte Vertragsstaaten : **BE CH DE FR GB IT LI NL**

(56) Entgegenhaltungen :
**FR-A- 1 162 853**
**FR-A- 2 390 449**
**GB-A- 831 346**

(73) Patentinhaber : **Richardson-Vicks, Inc.**
**Ten Westport Road**
**Wilton, Conn. 06897 (US)**

(72) Erfinder : **Hostettler, Hans Ulrich, Dr.**
**Weidenhofweg 28**
**CH-4144 Arlesheim (CH)**
Erfinder : **Pauling, Horst, Dr.**
**Ruchholzstrasse 39**
**CH-4103 Bottmingen (CH)**

(74) Vertreter : **Lederer, Franz, Dr. et al**
**Patentanwälte Dr. Franz Lederer Dipl.-Ing. Reiner F. Meyer-Roxlau Lucile-Grahn-Strasse 22**
**D-8000 München 80 (DE)**

**EP 0 049 498 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft organische Reste enthaltende Aluminiumverbindungen der allgemeinen Formel

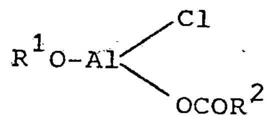

(I)

worin

$R^1$ $C_{2-18}$-Alkoxyalkyl, Phenoxy-$C_{1-6}$-alkyl oder mit $C_{2-6}$-Alkoxycarbonyl substituiertes Phenyl und

$R^2$ unsubstituiertes oder mit Halogen, $C_{1-4}$-Alkyl, $C_{1-4}$-Alkoxy, $C_{2-6}$-Alkanoyloxy und/oder $C_{2-6}$-Alkoxycarbonyl substituiertes Phenyl ; Phenyl-$C_{1-4}$-alkyl ; oder Pyridyl bedeuten.

Die Verbindungen der Formel I besitzen transpirationshemmende Wirkung und eignen sich insbesondere als Wirkstoffe für Antiperspirantien.

Die Erfindung betrifft ferner Verbindungen der Formel I als transpirationshemmende Mittel, Antiperspirantien, die als Wirkstoff mindestens eine Verbindung der Formel I enthalten, sowie die Verwendung solcher Antiperspirantien zur Hemmung der Transpiration.

Der in der obigen Definition der Formel I vorhandene Ausdruck « Alkyl » umfasst sowohl geradkettige als auch verzweigte Alkylgruppen. Dies gilt auch für den Alkylteil von Alkoxyalkyl, Phenoxyalkyl, mit Alkoxycarbonyl substituiertes Phenyl, mit Alkyl, Alkoxy, Alkanoyloxy und/oder Alkoxycarbonyl substituiertes Phenyl und Phenylalkyl. Der Ausdruck « Halogen » umfasst Fluor, Chlor Brom und Jod.

In den Verbindungen der Formel I sind in gewissen Fällen asymmetrische Kohlenstoffatome vorhanden, so dass solche Verbindungen als optische Antipoden vorliegen können. Die Formel I soll demnach diese möglichen isomeren Formen sowie die Racemate umfassen.

$R^1$ in der Bedeutung Phenoxy-$C_{1-6}$-alkyl ist vorzugsweise 2-Phenoxyäthyl.

Bedeutet $R^1$ mit $C_{2-6}$-Alkoxycarbonyl substituiertes Phenyl, so ist dies vorzugsweise 4-Aethoxycarbonylphenyl.

Bedeutet $R^2$ Phenyl-$C_{1-4}$-alkyl, so ist dies vorzugsweise Benzyl.

Hat $R^2$ die Bedeutung Pyridyl, so ist dies vorzugsweise 3-Pyridyl.

$R^1$ ist vorzugsweise Phenoxy-$C_{1-6}$-alkyl.

$R^2$ ist vorzugsweise substituiertes Phenyl oder Phenyl-$C_{1-4}$-alkyl.

Eine besonders bevorzugte Verbindung der Formel I ist Chlor-(2-phenoxy-äthoxy)-benzoato-aluminium.

Verbindungen der Formel I können dadurch hergestellt werden, dass man

a) eine Verbindung der allgemeinen Formel

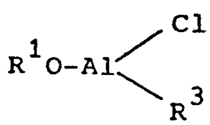

(II)

worin

$R^1$ die oben angegebene Bedeutung besitzt und

$R^3$ $C_{2-8}$-Alkyl bedeutet,

mit einer Säure der allgemeinen Formel

$$R^2COOH \qquad (III)$$

worin $R^2$ die oben angegebene Bedeutung besitzt, umsetzt,

b) eine Verbindung der allgemeinen Formel

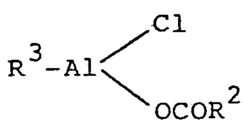

(IV)

worin $R^2$ und $R^3$ die oben angegebenen Bedeutungen besitzen,
mit einem Alkohol bzw. Phenol der allgemeinen Formel

$$R^1OH \qquad (V)$$

worin $R^1$ die oben angegebene Bedeutung besitzt, umsetzt,

c) eine Verbindung der allgemeinen Formel

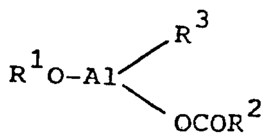 (VI)

worin $R^1$, $R^2$ und $R^3$ die oben angegebenen Bedeutungen besitzen, mit Chlorwasserstoff umsetzt,

d) eine Verbindung der allgemeinen Formel

$$(R^1O)_2AlCl \quad (VII)$$

worin $R^1$ die oben angegebene Bedeutung besitzt, mit einer Verbindung der allgemeinen Formel

$$(R^2COO)_2AlCl \quad (VIII)$$

worin $R^2$ die oben angegebene Bedeutung besitzt, umsetzt,

e) eine Verbindung der allgemeinen Formel

$$(R^1O)_3Al \quad (IX)$$

worin $R^1$ die oben angegebene Bedeutung besitzt, mit einer Verbindung der allgemeinen Formel

$$Al(OCOR^2)_3 \quad (X)$$

worin $R^2$ die oben angegebene Bedeutung besitzt, und mit Aluminiumtrichlorid umsetzt,

f) eine Verbindung der allgemeinen Formel

$$R^1OMe \quad (XI)$$

worin

$R^1$ die oben angegebene Bedeutung besitzt und

Me ein Alkalimetall, insbesondere Natrium oder Kalium, bedeutet,

mit einer Verbindung der allgemeinen Formel

$$MeOCOR^2 \quad (XII)$$

worin $R^2$ und Me die oben angegebenen Bedeutungen besitzen, und mit Aluminiumtrichlorid umsetzt, oder

g) eine Verbindung der allgemeinen Formel

$$(R^3)_2AlCl \quad (XIII)$$

worin $R^3$ die oben angegebene Bedeutung besitzt, mit einer Verbindung der Formel III und einer Verbindung der Formel V, wie oben definiert, umsetzt.

Als geeignete Lösungsmittel für die Durchführung der obigen sieben Verfahrensvarianten kommen nichtprotonische Lösungsmittel, insbesondere aliphatische, alicyclische oder aromatische Kohlenwasserstoffe wie n-Hexan, Cyclohexan und Benzol ; Aether und ätherartige Verbindungen wie Diäthyläther und Dioxan ; und halogenierte Kohlenwasserstoffe wie Methylenchlorid und Chlorbenzol in Frage.

Die Reaktionstemperaturen liegen zweckmässigerweise zwischen – 20 °C und 100 °C, vorzugsweise zwischen 0 °C und 50 °C. Im allgemeinen wird unter Normaldruck gearbeitet.

Da die Ausgangsmaterialien der Formeln II, IV, VI und XIII, d. h. jene Ausgangsmaterialien, die Aluminium-Kohlenstoff-Bindungen aufweisen, in der Regel sehr hydrolyse — empfindlich bzw. oxydationsempfindlich sind, werden die Reaktionen gemäss den Verfahrensvarianten a), b), c) und g) unter möglichst wasser- und luftfreien Bedingungen durchgeführt. Es werden daher zweckmässigerweise wasserfreie Lösungsmittel verwendet, und die Reaktionen werden zweckmässigerweise in trockener Schutzgasatmosphäre, z. B. unter Stickstoff oder Argon, durchgeführt.

Die Isolierung der so erhaltenen Verbindungen der Formel I erfolgt in üblicher Weise.

Die Varianten a) und b) sind bevorzugt.

Die als Ausgangsmaterialien verwendbaren Verbindungen der Formeln II, III, IV, V, VI, VII, VIII, IX, X, XI, XII und XIII sind entweder bekannt oder können nach an sich bekannten Methoden hergestellt werden.

Wie im Falle vieler bekannter Aluminiumverbindungen, kann das Aluminiumatom auch in den erfindungsgemässen Verbindungen der Formel I unter Zuhilfenahme von sogenannten Nebenvalenzbindungen Raumstrukturen ausbilden, bei denen das Aluminiumatom höhere Koordinationszahlen als 3, z. B. 4 oder 6, aufweist. Die entstehenden komplexen Strukturen weisen in der Regel kein einheitliches Molgewicht auf, so dass die Verbindungen je nach den sterischen oder elektronischen Gegebenheiten als Oligomere, z. B. Di-, Tri- oder Tetramere, oder sogar als Polymere vorliegen. Infolgedessen können die Verbindungen als Gemische mehrerer solcher Formen vorliegen, die entweder amorphe Festkörper mit oft hohen Schmelz- bzw. Zersetzungspunkten oder -intervallen oder glasig-harzige Substanzen sind. Aus diesem Grund sind die Verbindungen in vielen Fällen nicht destillierbar oder umkristallisierbar. Allerdings können, wie oben gesagt, bei Verwendung reiner Ausgangsmaterialien bereits analysenreine Verbindungen der Formel I oft erhalten werden.

Aus den obigen Gründen soll demgemäss die allgemeine Formel I nicht nur jene Verbindungen, die in monomerer Form vorliegen, sondern auch jene Verbindungen, die in oligomerer bzw. polymerer Form vorliegen, und Gemische solcher Verbindungen umfassen.

Die Verbindungen der Formel I besitzen, wie oben gesagt, transpirationshemmende Wirkung. Sie können demnach zu verschiedenartigen Mitteln formuliert werden. Die erfindungsgemässen Antiperspirantien sind dadurch gekennzeichnet, dass sie eine wirksame Menge mindestens einer Verbindung der allgemeinen Formel I, wie oben definiert, sowie in der Kosmetik gebräuchliches Trägermaterial bzw. anderweitig kosmetisch wirksames Begleitmittel enthalten. Solche Mittel liegen z. B. in Form von Pudern, Stiften, Gels, Crèmes, Lösungen, Sprays oder Aerosolen vor. Die Herstellung dieser Mittel erfolgt nach dem Fachmann bekannter Art und Weise.

Als Trägermaterialien für die erfindungsgemässen Antiperspirantien kommen die üblichen, in der Kosmetik für Antiperspirantien verwendeten Zusätze in Betracht, z. B. Lösungsmittel wie Alkohole, insbesondere Aethanol und Isopropanol, Wasser, Kohlenwasserstoffe, insbesondere n-Hexan und n-Heptan, chlorierte Kohlenwasserstoffe, insbesondere Methylenchlorid, und Aether und ätherartige Verbindungen, insbesondere Dimethyläther, Diäthyläther, Tetrahydrofuran, Dioxan und 6-Acetoxy-2,4-dimethyl-1,3-dioxan ; feste Trägerstoffe wie Talkum, mikrokristalline Cellulose und Stearylalkohol ; flüssige Trägerstoffe wie Isopropylmyristat und Silikonöl ; Dispergiermittel wie amorphes Siliziumdioxid und Magnesiumstearat ; Verdickungsmittel wie Cellulose-Polyalkyläther ; und Treibgase wie Aether, insbesondere Dimethyläther und tiefsiedende, verflüssigte, gegebenenfalls mit Halogen substituierte Alkane, insbesondere n-Propan, n-Butan, Isobutan, Dichlordifluormethan, Trichlorfluormethan und Dichlortetrafluoräthan sowie Mischungen hiervon.

Beispiele von anderweitig kosmetischen Begleitmitteln sind Mittel zur Erhöhung des Hautmetabolismus bzw. der Hautelastizität, wie Panthenol und dessen niedere Alkyläther, z. B. der Aethyläther ; bakterientötende Mittel wie quaternäre Ammonium-Verbindungen ; anderweitig transpirationshemmende Mittel ; desodorierende Mittel ; Parfüme.

In den erfindungsgemässen Antiperspirantien betragen die Konzentrationen an Wirkstoff bzw. Wirkstoffen der Formel I im allgemeinen 1 bis 30 Gewichtsprozent, vorzugsweise 2 bis 20 Gewichtsprozent.

Zur Herstellung der erfindungsgemässen Antiperspirantien wird mindestens ein Wirkstoff der Formel I mit dem benötigten Trägermaterial und eventuell mit dem anderweitig kosmetisch wirksamen Begleimittel vermischt. Das Vermischen kann einstufig oder mehrstufig und auf in der Kosmetik übliche Weise erfolgen.

Die erfindungsgemässen Antiperspirantien werden verwendet, indem man auf die zu behandelnde Fläche eine wirksame Menge des erfindungsgemässen Antiperspirans appliziert. Diese Applikation kann auf in der Kosmetik übliche Weise erfolgen, z. B. durch Einreiben oder Besprühen.

Aus der GB-A-831 346 sind verschiedene aluminoorganische Verbindungen bekannt, beispielsweise das Aluminiummonochlorid-mono-sek.-butoxid-mono-palmitat, von dem bekannt war (GB-A-806 182, FR-A-1 162 853), daß es auf dem Kosmetiksektor als Antitranspirans eingesetzt werden kann ; auch von anderen aus diesen Literaturstellen bekannten Aluminiumverbindungen wußte man, daß sie schweißhemmende Eigenschaften besitzen. Die erfindungsgemäßen Verbindungen werden durch diesen Stand der Technik nicht nahegelegt.

### Beispiele 1-3

Nach der Verfahrensvariante a) werden die entsprechenden Ausgangsmaterialien der Formeln II und III umgesetzt, um die in der nachstehenden Tabelle I aufgeführten Verbindungen der Formel I herzustellen.

(Siehe Tabelle I Seite 5 f.)

Tabelle I

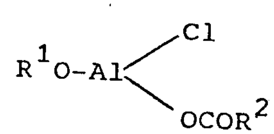

(I)

| Beispiel | Ausgangsmaterial der Formel II | Ausgangsmaterial der Formel III | Verbindung der Formel I $R^1$ | $R^2$ | Schmelzpunkt °C | Mikroanalyse (berechnet/gefunden) % C | % H | % Cl | % Al |
|---|---|---|---|---|---|---|---|---|---|
| 1 | Chlor-(2-phenoxyäthoxy)-isobutyl-aluminium | Benzoesäure | $C_6H_5OCH_2CH_2$ | $C_6H_5$ | 194° (unter Zersetzung) | 56,18<br>56,30 | 4,40<br>4,48 | 11,05<br>10,96 | —<br>— |
| 2 | Chlor-(2-methoxyäthoxy)-isobutyl-aluminium | Benzoesäure | $CH_3OCH_2CH_2$ | $C_6H_5$ | > 300° | 46,44<br>46,19 | 4,68<br>4,55 | —<br>— | 10,43<br>10,61 |
| 3 | Chlor-(p-äthoxycarbonyl-phenoxy)-isobutyl-aluminium | Benzoesäure | $p\text{-}C_2H_5OCOC_6H_5$ | $C_6H_5$ | ab 220° (unter Zersetzung) | 55,11<br>55,23 | 4,05<br>4,17 | 10,17<br>10,34 | —<br>— |

Formulierungsbeispiele :

## Beispiel 4

Ein Antiperspirans-Spray hat die folgende Zusammensetzung :

| | |
|---|---|
| Wirkstoff der Formel I | 6,00 g |
| Isopropylmyristat | 5,80 g |
| Amorphes Siliziumdioxid | 0,20 g |
| Treibgas, z. B. Gemisch aus Trichlorfluormethan und Dichlordifluormethan (60 : 40) | 88,00 g |

Die aus den obligen Bestandteilen bestehende Suspension des Wirkstoffes wird in eine metallische Aerosoldose eingefüllt.

## Beispiel 5

Ein Antiperspirans-Spray hat die folgende Zusammensetzung :

| | |
|---|---|
| Wirkstoff der Formel I | 5,00 g |
| Absoluter Aethanol | 20,00 g |
| Treibgas, z. B. Gemisch aus Trichlorfluormethan und Dichlordifluormethan (60 : 40) | 75,00 g |

Die aus den obigen Bestandteilen bestehende Lösung wird in eine gläserne Aerosolflasche eingefüllt.

## Beispiel 6

Ein Antiperspirans-Spray hat die folgende Zusammensetzung :

| | |
|---|---|
| Wirkstoff der Formel I | 5,00 g |
| Absoluter Aethanol | 95,00 g |
| Parfüm | beliebige Menge |

Die aus den obigen Bestandteilen bestehende Lösung wird in eine Handzerstäuberflasche aus Kunststoff eingefüllt.

## Beispiel 7

Ein Antiperspirans-Stift (wachsartig) hat die folgende Zusammensetzung :

| | |
|---|---|
| Wirkstoff der Formel I | 20,00 g |
| Stearylalkohol | 30,00 g |
| Silikonöl (Siedepunkt 190-200 °C) | 50,00 g |

Die obigen Bestandteile werden miteinander vermischt, geschmolzen und unter ständigem Rühren in Stifte gegossen.

## Beispiel 8

Ein Antiperspirans-Stift (trocken) hat die folgende Zusammensetzung :

| | |
|---|---|
| Wirkstoff der Formel I | 25,00 g |
| Talkum | 9,00 g |
| Mikrokristalline Cellulose | 65,75 g |
| Magnesiumstearat | 0,25 g |

Die obigen Bestandteile werden intensiv vermischt und durch ein isostatisches Pressverfahren zu Stiften geformt.

Beispiel 9

Ein Antiperspirans-Gel hat die folgende Zusammensetzung :

| | |
|---|---|
| Wirkstoff der Formel I | 5,00 g |
| Absoluter Aethanol | 94,00 g |
| Cellulose-Polyalkyläther | 1,00 g |

Die obigen Bestandteile werden vermischt, wobei sich das Gel bildet.
Biologischer Befund :

Beispiel 10

Die Prüfung der erfindungsgemässen Verbindungen der Formel I als Wirkstoffe für Antiperspirantien wird nach der Pads-Methode, die z. B. in J. Invest. Dermatol. *43,* 363-378 (1964), J. Soc. Cosmet. Chem. *23,* 22-43 (1972) und Acta Dermatovener (Stockholm), *55,* 241-260 (1975) ausführlich beschrieben ist, durchgeführt. Dabei wird eine Achselhöhle der Probanden (jeweils 1-13 Personen) mit 2 ml der geeigneten Anwendungsform (z. B. Lösung oder Spray bestimmter Wirkstoffkonzentration) des jeweiligen Wirkstoffs behandelt. Die andere Achselhöhle bleibt unbehandelt und dient als Kontrolle.
Das Ergebnis ist in der nachstehenden Tabelle II festgehalten :

Tabelle II

| Verbindung der Formel I (Beispiel Nr.) | Konzentration (Gewichtsprozent) und Lösungsmittel | Anzahl Probanden | Schweissreduktion (Durchschnittswert) |
|---|---|---|---|
| Chlor-(2-phenoxyäthoxy)-benzoatoaluminium (1) | 20 %ig in Feinsprit | 5 | 67,4 % |

**Ansprüche**

1. Verbindungen der allgemeinen Formel

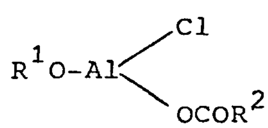

(I)

worin
$R^1$ $C_{2-18}$-Alkoxyalkyl, Phenoxy-$C_{1-6}$-alkyl oder mit $C_{2-6}$-Alkoxycarbonyl substituiertes Phenyl und
$R^2$ unsubstituiertes oder mit Halogen, $C_{1-4}$-Alkyl, $C_{1-4}$-Alkoxy, $C_{2-6}$-Alkanoyloxy und/oder $C_{2-6}$-Alkoxycarbonyl substituiertes Phenyl ; Phenyl-$C_{1-4}$-alkyl ; oder Pyridyl bedeuten.

2. Verbindungen nach Anspruch 1, worin $R^1$ Phenoxy-$C_{1-6}$-alkyl bedeutet.

3. Chlor-(2-phenoxy-äthoxy)-benzoato-aluminium.

4. Chlor-(2-methoxyäthoxy)-benzoato-aluminium.

5. Chlor-(p-äthoxycarbonyl-phenoxy)-benzoato-aluminium.

6. Verbindungen nach einem der Ansprüche 1 bis 5 als transpirationshemmende Mittel.

7. Antiperspirans, dadurch gekennzeichnet, daß es eine wirksame Menge mindestens einer der Verbindungen der Ansprüche 1 bis 5, sowie in der Kosmetik gebräuchliches Trägermaterial bzw. anderweitig kosmetisch wirksames Begleitmittel enthält.

8. Antiperspirans nach Anspruch 7, dadurch gekennzeichnet, daß es eine wirksame Menge Chlor-(2-phenoxyäthoxy)-benzoato-aluminium enthält.

9. Verfahren zur Hemmung der Transpiration, dadurch gekennzeichnet, daß man auf die zu behandelnde Fläche eine wirksame Menge eines der in den Ansprüchen 7 und 8 genannten Antiperspirantien appliziert.

10. Verwendung der in den Ansprüchen 1 bis 5 genannten Verbindungen zur Hemmung der Transpiration.

**Claims**

1. Compounds of the general formula

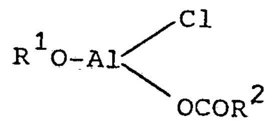

(I)

wherein

$R^1$ is $C_{2\text{-}18}$ alkoxyalkyl, phenoxy-$C_{1\text{-}6}$ alkyl or phenyl substituted by $C_{2\text{-}6}$ alkoxycarbonyl, and

$R^2$ is unsubstituted phenyl or phenyl substituted by halogen, $C_{1\text{-}4}$ alkyl, $C_{1\text{-}4}$ alkoxy, $C_{2\text{-}6}$ alkanoyloxy and/or $C_{2\text{-}6}$ alkoxycarbonyl, phenyl-$C_{1\text{-}4}$ alkyl or pyridyl.

2. Compounds according to claim 1, wherein $R^1$ is phenoxy-$C_{1\text{-}6}$ alkyl.

3. Chloro-(2-phenoxyethoxy)-benzoyloxy-aluminium.

4. Chloro-(2-methoxyethoxy)-benzoyloxy-aluminium.

5. Chloro-(p-ethoxycarbonylphenoxy)-benzoyloxy-aluminium.

6. Compounds according to one of claims 1 to 5, as anti-perspirants.

7. Anti-perspirant characterised in that it contains an effective amount of at least one of the compounds of claims 1 to 5 and a carrier conventional in cosmetics and further, as the case may be, an associated cosmetically-effective agent.

8. Anti-perspirant according to claim 7, characterised in that it contains an effective amount of chloro-(2-phenoxyethoxy)-benzoyloxy-aluminium.

9. A process for inhibiting perspiration, characterised in that an effective amount of the anti-perspirant defined in claims 7 and 8 is applied to the surface to be treated.

10. Use of compounds defined in claims 1 to 5, for the inhibition of perspiration.

**Revendications**

1. Composés de formule générale

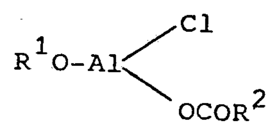

(I)

dans laquelle

$R^1$ représente un groupe alcoxyalkyle en $C_2$-$C_{18}$, un groupe phénoxy-alkyle en $C_1$-$C_6$ ou un groupe phényle substitué par alcoxycarbonyle en $C_2$-$C_6$ et

$R^2$ représente un groupe phényle substitué ou non par halogène, alkyle en $C_1$-$C_4$, alcoxy en $C_1$-$C_4$, alcanoyloxy en $C_2$-$C_6$ et/ou alcoxycarbonyle en $C_2$-$C_6$ ; un groupe phényl-alkyle en $C_1$-$C_4$ ; ou un groupe pyridyle.

2. Composés selon la revendication 1, dans lesquels $R^1$ représente un groupe phénoxy-alkyle en $C_1$-$C_6$.

3. Le chloro-(2-phénoxy-éthoxy)-benzoato-aluminium.

4. Le chloro-(2-méthoxy-éthoxy)-benzoato-aluminium.

5. Le chloro-(p-éthoxycarbonyl-phénoxy)-benzoato-aluminium.

6. Composés selon l'une des revendications 1 à 5 comme agents inhibant la transpiration.

7. Agent antisudoral, caractérisé en ce qu'il contient une quantité efficace d'au moins un des composés des revendications 1 à 5, ainsi qu'un matériau de support habituel en cosmétique ou un agent secondaire ayant une autre activité cosmétique.

8. Agent antisudoral selon la revendication 7, caractérisé en ce qu'il contient une quantité efficace de chloro-(2-phénoxy-éthoxy)-benzoato-aluminium.

9. Procédé pour inhiber la transpiration, caractérisé en ce que l'on applique sur la surface à traiter une quantité active d'un des agents antisudoraux mentionnés dans les revendications 7 et 8.

10. Utilisation des composés mentionnés dans les revendications 1 à 5 pour inhiber la transpiration.